# EUROPEAN PATENT SPECIFICATION

(11) **EP 3 435 092 B1**
(45) Date of publication and mention of the grant of the patent: **06.08.2025**
(21) Application number: 17769663.0
(22) Date of filing: 31.01.2017
(51) Int. Cl.: G01N 35/02, G01N 35/04, G01N 35/00

(54) **SPECIMEN CONVEYANCE SYSTEM AND SPECIMEN CONVEYANCE METHOD**
PROBENBEFÖRDERUNGSSYSTEM UND PROBENBEFÖRDERUNGSVERFAHREN
SYSTÈME DE TRANSPORT D'ÉCHANTILLON ET PROCÉDÉ DE TRANSPORT D'ÉCHANTILLON

(30) Priority: 24.03.2016 JP 2016060882
(43) Date of publication of application: 30.01.2019
(73) Proprietor: Hitachi High-Tech Corporation, Minato-ku Tokyo 105-6409 (JP)
(72) Inventor: SHIMODA Akihiro, Tokyo 105-6409 (JP); YANO Shigeru, Tokyo 105-6409 (JP)
(74) Representative: Strehl & Partner mbB
(86) International application number: PCT/JP2017/003482
(87) International publication number: WO 2017/163616

(56) References cited:
- EP-A1- 2 354 797
- EP-A1- 2 485 057
- EP-A1- 2 894 479
- JP-A- 2000 081 439
- JP-A- 2001 228 160
- JP-A- 2010 107 383
- JP-A- 2010 156 602
- JP-A- 2011 158 394
- JP-A- 2012 093 313
- JP-A- H0 943 249
- US-A- 6 117 683
- US-A1- 2012 109 531

## Description

### Technical Field

The present invention relates to a specimen conveyance system and a specimen conveyance method.

### Background Art

In a specimen examination pre-treatment system, according to specimen examination request information, a pre-treatment such as centrifugal separation or dispensing is performed on a specimen such as blood or urine and the pre-treated specimen is conveyed to an analysis device that examines the specimen by the specimen conveyance system. The specimen examination request information that the specimen pre-treatment system receives from a host computer contains information regarding an analysis device or a stocker to which a specimen is to be conveyed for each examination item.

In the case of specimen examination request information containing plural examination items, there are a plurality of analysis devices or stockers where a specimen is to be conveyed. In this case, priorities are given to the analysis devices or stockers where a specimen is to be conveyed, and thus the specimen is conveyed according to the priorities.

The order of conveyance to the analysis devices or stockers is determined when a specimen is loaded on the specimen pre-treatment system, and the order of conveyance is not changed during a pre-treatment unless error occurs during the treatment on the specimen.

Therefore, when it is necessary to reexamine the specimen as a result of the analysis in the analysis device, the pre-treatment system cannot process the additional request information even when additional request information is received from the host computer, because the specimen is treated according to the order of conveyance determined at the time of loading of the specimen.

In order to process the additional request information, it is necessary to reload the specimen (specimen that is required to be reexamined) that is accommodated in a specimen storage unit of the specimen pre-treatment system after all the conveyance processes determined at the time of loading of the specimen that is required to be reexamined are completed.

When additional request information is received after loading of a specimen, an example of a method of changing a conveyance route in consideration of the additional request information is disclosed in PTL 1.

### Citation List

### Patent Literature

PTL 1: JP-A-2012-093313
EP 2 894 479 A1 discloses an automatic analyzer having a speciman conveyance system with the features in the pre-characterizing portion of Claim 1. Further related specimen transport systems are disclosed in US 6 117 683 A and EP 2 354 797 A1.

### Summary of Invention

### Technical Problem

However, in the method described in PTL 1, a specimen is loaded and conveyed to an analysis device, and moves in one direction until the specimen is accommodated in a storage unit. Therefore, once a specimen stops by an analysis device, the specimen cannot stop by the analysis device again.

Therefore, when additional request information is received from the host computer during a treatment of a specimen, the specimen conveyance system cannot process the additional request information.

Accordingly, it is necessary to reload the specimen for examination according to the additional request information after a person checks the fact that incomplete examination items are present. As a result, loss of examination time occurs. Alternatively, there may be a case where a person cannot check incomplete examination items, which may lead to a delay of examination result reporting.

The object of the present invention is to realize a specimen conveyance system and a specimen conveyance method in which a specimen conveyance route can be reset even when additional request information is received after loading of a specimen.

### Solution to Problem

In order to achieve the object, the present invention is configured by the specimen conveyance system and the specimen conveyance method defined in the independent claims. Further advantageous feature are set out in the dependent claims.

### Advantageous Effects of Invention

It is possible to realize a specimen conveyance system and a specimen conveyance method in which a specimen conveyance route can be reset even when additional request information is received after loading of a specimen.

### Brief Description of Drawings

[Fig. 1] Fig. 1 is a diagram illustrating a schematic configuration of a specimen conveyance system according to one embodiment of the present invention.
[Fig. 2] Fig. 2 is a diagram describing a process when a specimen ID cannot be read when a specimen is returned from an analysis device by a transfer machine in the embodiment of the present invention.
[Fig. 3] Fig. 3 is a block diagram illustrating an internal configuration of an operation control unit.
[Fig. 4] Fig. 4 is a diagram describing the summary of a process flow of a device communication process unit of the operation control unit.
[Fig. 5] Fig. 5 is a diagram illustrating a process flow of a host communication process unit.
[Fig. 6] Fig. 6 is a diagram illustrating a process flow of a conveyance route generating unit.
[Fig. 7] Fig. 7 is a diagram illustrating an example of a specimen conveyance system different from the present invention for comparison to the present invention.

### Description of Embodiments

An embodiment of the present invention will be described with reference to the accompanying drawings.

### Embodiment

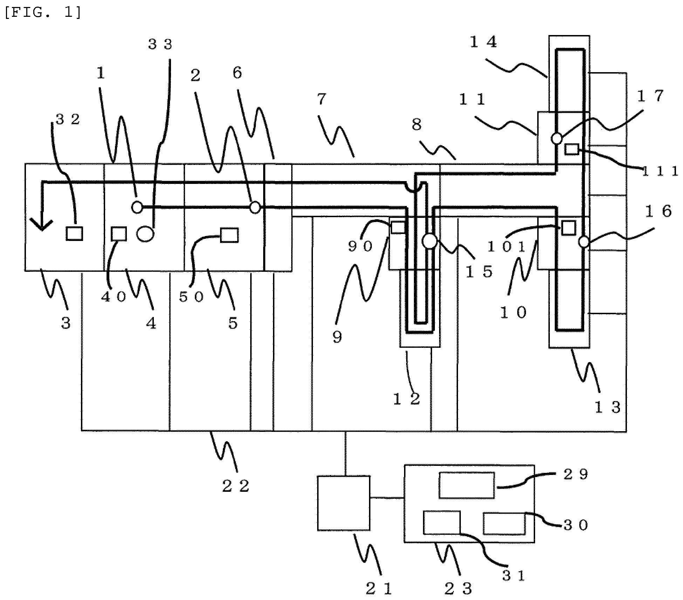

Fig. 1 is a diagram illustrating a schematic configuration of a specimen conveyance system according to one embodiment of the present invention.

In Fig. 1, the specimen conveyance system includes a storage unit (collecting unit) 3, a loading unit 4, a dispensing unit 5, a conveyance line 6, a conveyance line 7, a conveyance line 8, a transfer machine 9, a transfer machine 10, a transfer machine 11, an operation control unit (computer) 21, and a host computer 23. The operation control unit 21 controls operations and the like of the storage unit 3, the loading unit 4, the dispensing unit 5, the conveyance line 6, the conveyance line 7, the conveyance line 8, the transfer machine 9, the transfer machine 10, and the transfer machine 11. An information reading device 32 such as a bar code reader is arranged in the storage unit 3.

Likewise, in the invention, an information reading device 40 is arranged in the loading unit 4, an information reading device 50 is arranged in the dispensing unit 5, an information reading device 90 is disposed in the transfer machine 9, an information reading device 101 is arranged in the transfer machine 10, and an information reading device 111 is arranged in the transfer machine 11.

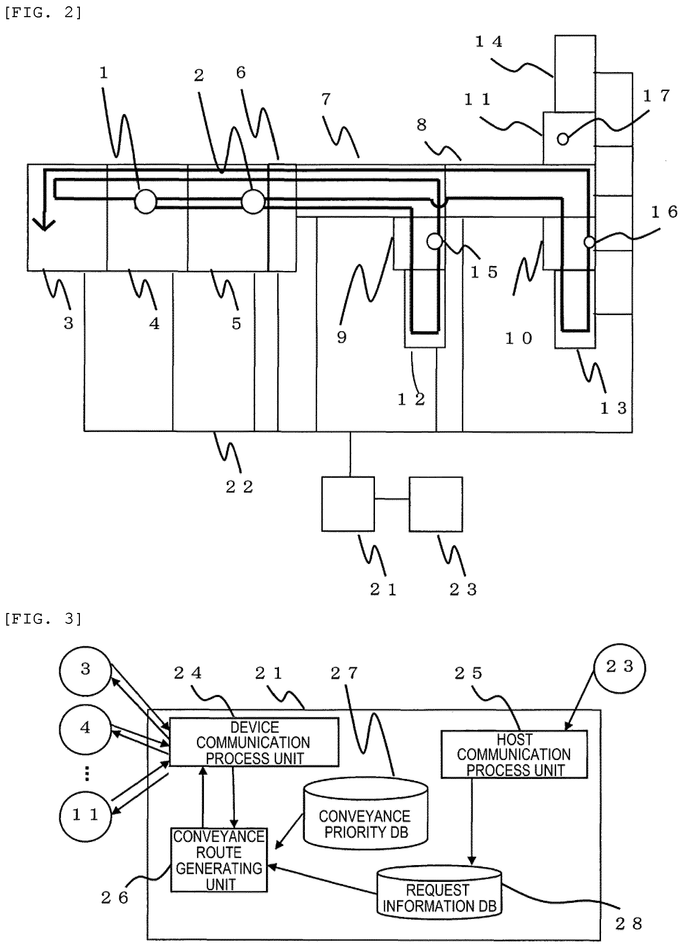

An internal configuration of the operation control unit 21 will be described. Fig. 3 is a block diagram illustrating the internal configuration of the operation control unit 21.

In Fig. 3, the operation control unit 21 includes a device communication process unit 24, a host communication process unit 25, a conveyance route generating unit 26, a conveyance priority DB (conveyance priority storage unit) 27, and a request information DB (request information storing unit) 28.

Each of the storage unit 3, the loading unit 4, the dispensing unit 5, the conveyance line 6, the conveyance line 7, the conveyance line 8, the transfer machine 9, the transfer machine 10, and the transfer machine 11 exchanges information by the device communication process unit 24 of the operation control unit 21 through a communication cable 22.

In addition, the host communication process unit 25 of the operation control unit 21 exchanges information with the host computer 23 through the communication cable 22. Here, the host computer 23 includes a control unit (CPU) 29, a storage unit 30, and an input/output unit 31.

A specimen is accommodated in a specimen container 33, and the specimen container 33 is mounted on a specimen container holder.

Before being conveyed to the conveyance lines 6, 7, and 8, the holder where the specimen container 33 is mounted stops by the transfer machine 9, 10, and 11 along the conveyance lines 6, 7, and 8 according to stop-by information (conveyance route) set by the operation control unit 21 such that treatments are performed by analysis devices 12, 13, and 14 at stop-by positions. The analysis device 14 may be a stocker. In addition, the conveyance lines 6, 7, and 8 can convey the specimen container 33 in both directions.

The specimen container 33 that accommodates the specimen loaded from the loading unit 4 is transferred to the specimen container holder, a specimen ID of the specimen is read by the information reading device, the specimen container 33 waits at a request reception point 1, and the loading unit 4 reports the stand-by to the operation control unit 21.

The operation control unit 21 receiving the report queries the host computer 23 for request information based on the specimen ID of the specimen by the host communication process unit 25. The host computer 23 reports the request information of the specimen to the operation control unit 21 by the host communication process unit 25. The operation control unit 21 determines a conveyance route based on the request information received at this time.

The request information received by the operation control unit 21 will be described as a request that causes the specimen to stop by the analysis device 12 and causes the specimen container of the specimen to be accommodated in the storage unit 3.

The operation control unit 21 stores the request information received from the host computer 23 in the request information DB 28. The conveyance route generating unit 26 generates a conveyance route using the conveyance priority DB 27 and the request information DB 28. Next, the specimen is transferred from the loading unit 4 to the dispensing unit 5, a dispensing treatment such as dispensing from a main specimen to a sub specimen is performed in the dispensing unit 5, and the presence of additional request information (additional examination item for the specimen) is checked at a destination determination point 2. At this point, when the additional request information is not received, the conveyance route is not changed. At this time, the additional request information is not received.

The specimen is held in the specimen container holder, is conveyed to the transfer machine 9 connected to the conveyance line 7 and the analysis device 12 through the conveyance lines 6 and 7, is conveyed into the analysis device 12 by the transfer machine 9, and is analyzed by the analysis device 12.

The analysis device 12 where the specimen arrives reports the arrival of the specimen to the operation control unit 21 through the communication cable 22. The operation control unit 21 determines whether or not a treatment is necessary in the analysis device 12, and instructs the analysis device 12 to perform the treatment content. The analysis device 12 performs the treatment according to the instruction of the operation control unit 21, and reports the treatment result to the operation control unit 21. The operation control unit 21 receiving the report instructs to convey the specimen to the next stop-by device based on the treatment result and the conveyance route generated at the time of loading of the specimen.

This way, the specimen loaded from the loading unit 4 is conveyed to the analysis device 12 through the dispensing unit 5, the conveyance line 6, the conveyance line 7, and the transfer machine 9, and is conveyed into the transfer machine 9 from the analysis device 12. The specimen ID of the returned specimen is read by the information reading device 90, and the specimen is conveyed out to the conveyance line 7 and is collected to be accommodated in the storage unit 3 through the conveyance line 6, the dispensing unit 5, and the loading unit 4. At this time, before the specimen is accommodated in the storage unit 3, the specimen ID of the specimen is read by the information reading device 40 of the loading unit 4 to check whether or not an abnormality is found.

At this time, when an abnormality is found in the analysis result, the operation control unit 21 receives the additional request information from the host computer 23.

When the analysis device 12 performs the treatment according to the instruction of the operation control unit 21, and an abnormality is found in the analysis result, the analysis device 12 notifies the host computer 23 of the fact that the abnormality is found (notification of abnormality result information), through the operation control unit 21. The host computer 23 generates additional request information based on the notified abnormality result information to notify to the operation control unit 21. The operation control unit 21 stores the notified additional request information in the request information DB 28.

At this time, the additional request information that has been received from the host computer 23 by the operation control unit 21 is a request that causes the specimen to stop by the analysis device 12, the analysis device 13, and the analysis device 14 and to be accommodated in the storage unit 3.

In the analysis device 12, the abnormality is found in the analysis result of the specimen. However, depending on the type of the analysis device, a countermeasure against the abnormality can be taken, and after the specimen is conveyed to another analysis device 13 or 14 and analyzed, the abnormality countermeasure process can be terminated in the analysis device 12. For example, when dispensing of a reagent fails, the dispensing of the reagent can be accurately performed by cleaning a reagent nozzle.

When the specimen returns from the analysis device 12 to the transfer machine 9, the specimen ID of the specimen is read by the information reading device 90, and the read specimen ID is notified to the operation control unit 21. Next, when the specimen is transferred to a destination determination point 15, the operation control unit 21 checks the request information DB 28 and receives the additional request information. Therefore, the operation control unit 21 regenerates a route.

At this time, conveyance priorities managed by the conveyance priority DB 27 are the analysis device 13, the analysis device 14, and the analysis device 12 in order from the highest to the lowest. Therefore, the conveyance route of the specimen generated at the destination determination point 15 is a route along which the specimen is conveyed to the storage unit 3 through the analysis device 13, the analysis device 14, and the analysis device 12.

The specimen is conveyed from the transfer machine 9 into the transfer machine 10 connected to the conveyance line 8 and the analysis device 13 through the conveyance lines 7 and 8, and then is conveyed into the analysis device 13. The conveyed specimen is analyzed by the analysis device 13. When the analysis in the analysis device 13 ends, the specimen is conveyed out from the analysis device 13 and returns to the transfer machine 10, and the specimen ID thereof is read by the information reading machine 101. Next, at a destination determination point 16, the operation control unit 21 checks the request information DB 28 in response to the notification from the transfer machine 10.

At this time, the additional request information for the specimen is set not to be present. Therefore, the specimen is conveyed from the destination determination point 15 to the transfer machine 11 connected to the conveyance line 8 and the analysis device 14 through the conveyance line 8. The specimen is conveyed into the analysis device 14 and analyzed.

The specimen is analyzed by the analysis device 14, is conveyed out from the analysis device 14, and returns to the transfer machine 11. The specimen ID of the specimen is read by the information reading device 111.

Next, at a destination determination point 17, when the notification is given to the operation control unit 21, the operation control unit 21 checks the request information DB 28. Even at this time, the additional request information for the specimen is set not to be present. Therefore, the specimen is conveyed from the transfer machine 11 to the transfer machine 9 through the conveyance line 8. The specimen is conveyed again from the transfer machine 9 to the analysis device 12 and analyzed.

When the specimen is analyzed by the analysis device 12, the specimen returns from the analysis device 12 to the transfer machine 9, and the specimen ID of the specimen is read by the information reading device 90. When the specimen is conveyed to the destination determination point 15, the operation control unit 21 checks the request information DB 28. Even at this time, the additional request information is set not to be present. Therefore, the specimen is conveyed from the transfer machine 90 to the storage unit 3 through the conveyance lines 7 and 6, the dispensing unit 5, and the loading unit 4.

The specimen ID is read by the information reading device of the storage unit 3 to check whether or not an abnormality is found, and then the specimen is accommodated in the storage unit 3.

Fig. 2 is a diagram illustrating a progress when an abnormality is found in the analysis result of the analysis device 12 and the specimen ID cannot be read when the specimen returns from the analysis device 12 to the transfer machine 9 in the embodiment. For simplicity, Fig. 2 omits the information reading device illustrated in Fig. 1.

In Fig. 2, as in the example illustrated in Fig. 1, after the specimen ID is read, the specimen container 33 loaded from the loading unit 4 waits at the request reception point 1, and the loading unit 4 reports the waiting to the operation control unit 21. Request information that causes the specimen to stop by the analysis device 12, to be analyzed, and to be accommodated in the storage unit 3 is received from the host computer 23 by the operation control unit 21, and is stored in the request information DB 28.

The conveyance route generating unit 26 of the operation control unit 21 generates a conveyance route using the conveyance priority DB 27 and the request information DB 28. Next, the specimen is dispensed by dispensing unit 5, and the presence of additional request information is checked at the destination determination point 2. At this point, when the additional request information is not received, the conveyance route is not changed.

The specimen is conveyed to the analysis device 12 through the conveyance lines 6 and 7 and the transfer machine 9 and analyzed. At this time, when an abnormality is found in the analysis result, the analysis device 12 notifies the host computer 23 of the fact that the abnormality is found in the analysis result through the communication cable 22 and the operation control unit 21.

The host computer 23 transmits the additional request information to the operation control unit 21. At this time, the additional request information that has been received by the operation control unit 21 is a request that causes the sample to stop by the analyzer 13, to be analyzed, and to be accommodated in the housing unit 3.

When the specimen returns from the analysis device 12 to the transfer machine 9, the specimen ID of the specimen is read by the information reading device 90. When the specimen ID cannot be read due to contamination or the like, the fact is notified to the operation control unit 21 at the destination determination point 15 of the specimen, and the operation control unit 21 generates a conveyance route to the loading unit 4 and notifies the generated conveyance route to the transfer machine 9. The transfer machine 9 conveys the specimen to the conveyance line 7 and conveys the specimen to the loading unit 4 through the conveyance line 6 and the dispensing unit 5.

In the loading unit 4, the specimen ID of the specimen is read by the information reading device 40, and notified to the operation control unit 21. The specimen waits at the request reception point 2, and the request information DB 28 is checked through the operation control unit 21.

The operation control unit 21 receives the additional request information, and thus regenerate a route. At this time, the conveyance route of the specimen generated at the destination determination point 2 is a route where the specimen is conveyed to the analysis device 13 and analyzed and then is conveyed to the storage unit 3 through the conveyance lines 8, 7, and 6, the dispensing unit 5, and the loading unit 4.

The specimen is conveyed to the analysis device 13 through the conveyance lines 6, 7, and 8 and the transfer machine 10 and is analyzed by the analysis device 13. After the analysis of the specimen by the analysis device 13, the specimen returns from the analysis device 13 to the transfer machine 10, the specimen ID is read, and the request information DB 28 of the operation control unit 21 is checked at the destination determination point 16.

At this time, the additional request information for the specimen is not present. Therefore, the specimen is conveyed to the storage unit 3 through the conveyance lines 8, 7, and 6, the dispensing unit 5, and the loading unit 4 according to the route generated at the destination determination point 2. In the storage unit 3, the specimen ID of the specimen is read, the fact that an abnormality is not present is checked, and the specimen is accommodated.

As in the example illustrated in Fig. 2, when an abnormality is found in the analysis result of the analysis device 12 and the specimen ID cannot be read in the transfer machine 9, the specimen is conveyed to the loading unit 4 instead of being conveyed to the storage unit 3, the specimen ID is read, and the specimen is conveyed to the analysis device and analyzed according to the conveyance route generated by the operation control unit 21. As a result, even when an abnormality is found in the analysis result of the analysis device 12 and the specimen ID cannot be read in the transfer machine 9, the specimen can be rapidly analyzed, and the specimen analysis time can be reduced.

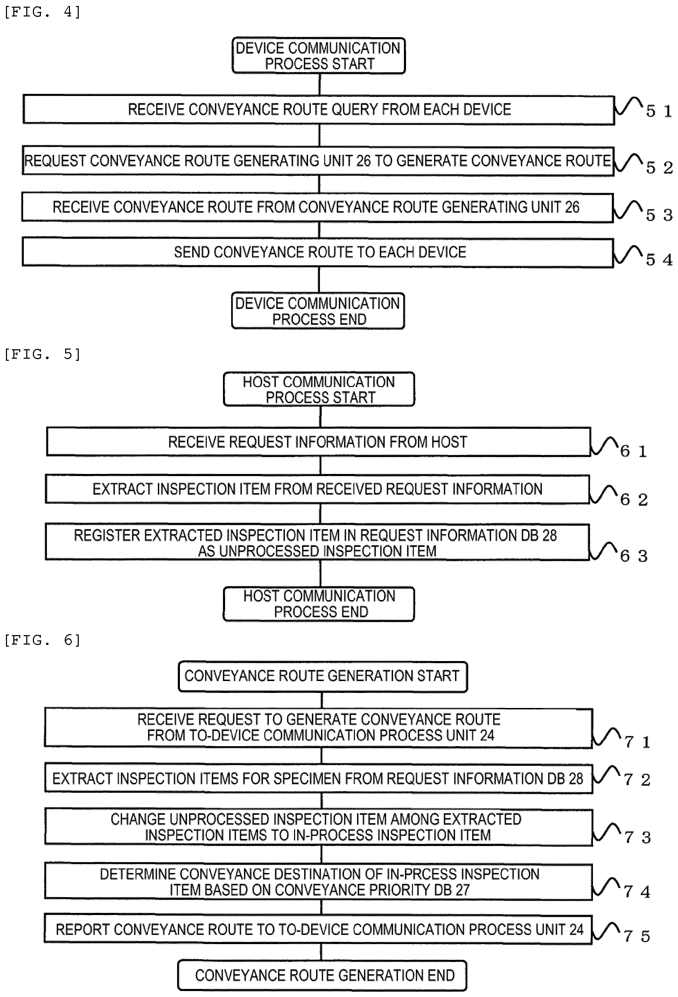

Fig. 4 is a diagram describing the summary of a process flow of the device communication process unit 24 of the operation control unit 21 in the embodiment.

In Fig. 4, when a communication process between the loading unit 4 or the like and the device communication process unit 24 starts, the communication process unit 24 receives a conveyance route query of the specimen from each of the devices and checks a process status of the specimen and error occurrence (process 51).

The conveyance route generating unit 26 is requested to generate a conveyance route of the specimen (process 52).

The device communication process unit 24 receives the generated conveyance route from the conveyance route generating unit 26 (process 53) and instructs the conveyance route to each device (process 54).

The device communication process of the device communication process unit 24 ends.

Fig. 5 is a diagram describing the summary of a process flow of the host communication process unit 25 in the embodiment.

In Fig. 5, request information is received from the host computer 23 when the host communication process unit 25 analyzes the received request information (process 61). An examination item is extracted from the analyzed request information (process 62). The extracted examination item is registered in the request information DB 28 as an unprocessed examination item (process 63). As a result, the host communication process of the host communication process unit 25 ends.

Fig. 6 is a diagram describing the summary of a process flow of the conveyance route generating unit 26 in the embodiment.

In Fig. 6, the conveyance route generating unit 26 checks a process status of the specimen when receiving a request to generate a conveyance route from the device communication process unit 24 (process 71). Next, the conveyance route generating unit 26 extracts examination items for the specimen from the request information DB 28 (process 72). Only unprocessed examination items among the extracted examination items are changed into in-process examination items (process 73) .

Next, the order of conveyance of the specimen is determined based on a conveyance destination assigned to each in-process examination item and information stored in the conveyance priority DB 27 (process 74). The conveyance route generating unit 26 reports the conveyance route to the device communication process unit 24 according to the determined order of conveyance (process 75). As a result, the conveyance route generating process of the conveyance route generating unit 26 ends.

Next, an example different from the present invention will be described for comparison to the present invention.

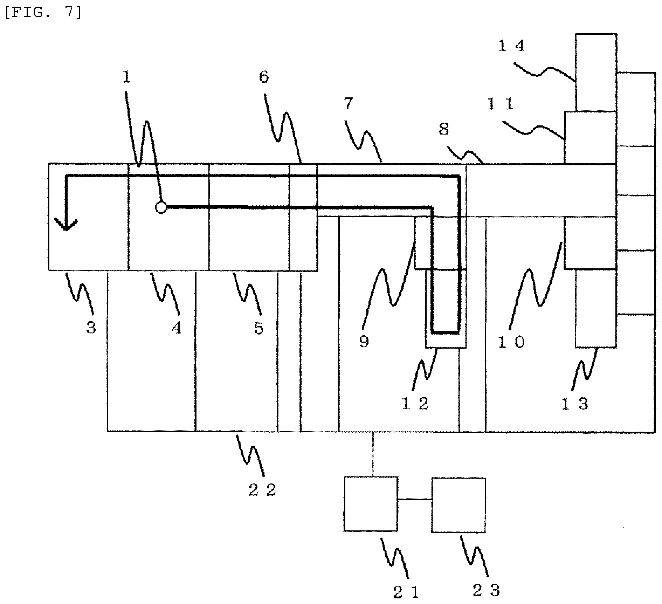

Fig. 7 is a block diagram illustrating a specimen conveyance system according to an example not forming part of, but useful for understanding the present invention.

A difference between the example of Fig. 7 and the example illustrated in Fig. 1 that is the embodiment of the present invention is: in the embodiment of the present invention, the abnormality result of the analysis in each analysis device 12, 13, and 14 is notified to the operation control unit 21 and the host computer 23, the generated additional request information is checked at the destination determination points 15, 16, and 17, and the subsequent conveyance route is determined.

In the example of Fig. 7, once the conveyance route of the specimen is determined, the conveyance route cannot be changed unless the specimen is conveyed from the analysis device 12, 13, or 14 to the storage unit 3.

The specimen conveyance system illustrated in Fig. 7 is controlled by the operation control unit 21 as in the example of Fig. 1. Each of the storage unit 3, the loading unit 4, the dispensing unit 5, the conveyance line 6, the conveyance line 7, the conveyance line 8, the transfer machine 9, the transfer machine 10, and the transfer machine 11 constituting the specimen conveyance system exchanges information with the operation control unit 21 through the communication cable 22.

In addition, the operation control unit 21 exchanges information with the host computer 23 through the communication cable 22. The specimen container holder where the specimen is mounted stops by a stop-by position according to stop-by information defined by the operation control unit 21, and the specimen is treated by a device at the stop-by position.

The specimen loaded from the loading unit 4 is transferred to the detection container holder and the specimen ID of the specimen is read. The specimen container holder waits at the request reception point 1, and the loading unit 4 reports the stand-by to the operation control unit 21. The operation control unit 21 receiving the report queries the host computer 23 for request information based on the specimen ID of the specimen.

The host computer 23 reports the request information of the specimen to the operation control unit 21. The operation control unit 21 determines a conveyance route based on the request information received at this time. The request information received at this time is a request that causes the sample to stop by the analyzer 12, to be analyzed, and to be accommodated the specimen in the housing unit 3.

Each device where the specimen arrives reports the arrival of the specimen to the operation control unit 21. The operation control unit 21 determines the necessity of a treatment in each device, and instructs each device to perform the treatment contents. Each device performs the treatment according to the instruction of the operation control unit 21, and reports the treatment result to the operation control unit 21.

The operation control unit 21 receiving the report instructs to convey the specimen to the next stop-by device based on the treatment result and the conveyance route generated at the time of loading of the specimen.

This way, the specimen loaded from the loading unit 4 is conveyed to the analysis device 12 through the dispensing unit 5, the conveyance line 6, the conveyance line 7, and the transfer machine 9 and is analyzed.

The specimen ID of the specimen returning from the analysis device 12 to the transfer machine 9 is read, and then the specimen is accommodated in the storage unit 3 through the conveyance line 7, the conveyance line 6, the dispensing unit 5, and the loading unit 4.

At this time, before the specimen is accommodated in the storage unit 3, the specimen ID of the specimen is read to check whether or not an abnormality is found.

In the example illustrated in Fig. 7, even when additional request information is received by the analysis device 12, the conveyance route cannot be changed until the specimen is accommodated in the storage unit 3 according to the route of the transfer machine 9, the analysis device 12, and the storage unit 3, which is the determined conveyance route, after loading of the specimen from the loading unit 4.

Therefore, in the example of Fig. 7, whenever the analysis abnormality result of the specimen is detected in the analysis device, an operation of returning the specimen from the analysis device to the storage unit 3 is necessary in each time, and a long period of time is required for the analysis of the specimen.

On the other hand, in the embodiment of the present invention, when an abnormality is found in the analysis result of the specimen in the analysis device analyzing the specimen, the operation control unit 21 regenerates a conveyance route of the specimen based on the information (for example, the occurrence of the abnormality or the kind of the abnormality) regarding the abnormality in the analysis result of the specimen, and the specimen is conveyed according to the regenerated conveyance route.

As a result, the specimen having the abnormality result can be conveyed to an appropriate analysis device without passing through the storage unit 3, and thus the specimen analysis time can be reduced.

In addition, a priority is given to a dispensing sub specimen container based on a request item requested for the specimen, and dispensing is performed according to the priority. As a result, when the volume of a main specimen is insufficient compared to the total dispensing volume, the risk of a delay of examination result reporting can be reduced.

As described above, according to the present invention, it is possible to realize a specimen conveyance system and a specimen conveyance method in which a specimen conveyance route can be reset even when additional request information is received after loading of a specimen.

All the operations by the host computer 23 can also be configured to be executed by the operation control unit 21.

### Reference Signs List

1: request reception point
2, 15, 16, 17: destination determination point
3: storage unit
4: loading unit
5: dispensing unit
6, 7, 8: conveyance line
9, 10, 11: transfer machine
12, 13, 14: analysis device
21: operation control unit
22: communication cable
23: host computer
24: device communication process unit
25: host communication process unit
26: conveyance route generating unit
27: conveyance priority DB (conveyance priority storage unit)
28: request information DB (request information storage unit)
29: control unit
30: storage unit
31: input/output unit
32, 40, 50, 90, 101, 111: information reading device
33: specimen container

## Claims

1. A specimen conveyance system comprising:
a loading unit (4) for loading a specimen;
a conveyance line (6-8) for conveying the specimen in both directions;
a plurality of analysis devices (12-14) that are connected to the conveyance line and adapted to analyze the specimen based on examination items of the specimen;
a plurality of transfer machines (9-11), each connected to a corresponding one of the plurality of analysis devices and to the conveyance line and adapted to convey the specimen out from the conveyance line into the analysis device and out from the analysis device into the conveyance line,
a storage unit (3) for collecting and accommodating the specimen; and
an operation control unit (21) for setting a conveyance route of the specimen before the specimen is conveyed through the conveyance line,
wherein, when an additional examination item is requested for the specimen after it has been conveyed from one of the analysis devices into the corresponding transfer machine, the operation control unit is adapted to reset the conveyance route of the specimen such as to include an analysis device that can analyze the additional examination item and such that the specimen is conveyed according to the reset conveyance route;
**characterized in that**
the specimen conveyance system further comprises information reading devices (40, 50, 90, 101, 111) adapted to read the specimen information of the specimen and arranged in each of the loading unit (4), a dispensing unit (5), and the transfer machines (9-11);
when an abnormality is found in an analysis result of the specimen at any one of the plurality of analysis devices (12-14), the analysis device is adapted to notify the abnormality result to the operation control unit (21), and the operation control unit is adapted to reset the conveyance route of the specimen based on the notified abnormality result and based on specimen information read by the information reading device arranged in the transfer machine connected to the analysis device such that the specimen, after it has been conveyed into the transfer machine from the analysis device, is conveyed according to the reset conveyance route; and,
when the specimen information of the specimen having the abnormality result and having been conveyed into the transfer machine from the analysis device cannot be read by the information reading device arranged in the transfer machine, the operation control unit is adapted to convey the specimen having the abnormality result from the transfer machine to the loading unit through the conveyance line, to read the specimen information of the specimen having the abnormality result by the information reading device arranged in the loading unit, and to set the conveyance route of the specimen based on the read specimen information.

2. A specimen conveyance method comprising:
reading specimen information of a specimen loaded from a loading unit (4) with an information reading device (40);
setting a conveyance route of the specimen based on the read specimen information with an operation control unit (21);
conveying the specimen to one or more of a plurality of analysis devices (12-14) through a conveyance line (6-8) via a corresponding one of a plurality of transfer machine (9-11) connected to each analysis device and to the conveyance line according to the set conveyance route to analyze the specimen;
resetting a conveyance route of the specimen such as to include an analysis device that can analyze an additional examination item, when the additional examination item is requested for the specimen after it has been conveyed from the analysis device into the corresponding transfer machine after the analysis of the specimen by the analysis device; and
conveying the specimen according to the reset conveyance route;
wherein, when an abnormality is found in an analysis result of the specimen at any one of the plurality of analysis devices (12-14), the analysis device notifies the abnormality result to the operation control unit (21), and the operability control unit resets the conveyance route of the specimen based on the notified abnormality result and based on specimen information read by an information reading device (90, 101, 111) arranged in the transfer machine connected to the analysis device such that the specimen, after it has been conveyed into the transfer machine (91-11) from the analysis device, is conveyed according to the reset conveyance route; and
when the specimen information of the specimen having the abnormality result and having been conveyed into the transfer machine from the analysis device cannot be read by the information reading device arranged in the transfer machine, the operation control unit conveys the specimen having the abnormality result from the transfer machine to the loading unit through the conveyance line, reads the specimen information of the specimen having the abnormality result by the information reading device arranged in the specimen loading unit, and sets a conveyance route of the specimen based on the read specimen information.

## Patentansprüche

1. Probenbeförderungssystem, umfassend:
eine Ladeeinheit (4) zum Laden einer Probe;
eine Beförderungslinie (6-8) zum Befördern der Probe in beide Richtungen;
mehrere Analysevorrichtungen (12-14), die mit der Beförderungslinie verbunden sind und dazu ausgelegt sind, die Probe basierend auf Untersuchungselementen der Probe zu analysieren;
mehrere Transfermaschinen (9-11), deren jede mit einer entsprechenden der mehreren Analysevorrichtungen und mit der Beförderungslinie verbunden ist und die jeweils dazu ausgelegt sind, die Probe aus der Beförderungslinie in die Analysevorrichtung und aus der Analysevorrichtung in die Beförderungslinie zu befördern,
eine Speichereinheit (3) zum Sammeln und Aufnehmen der Probe; und
eine Betriebssteuereinheit (21) zum Einstellen einer Beförderungsroute der Probe, bevor die Probe über die Beförderungslinie befördert wird,
wobei, wenn ein zusätzliches Untersuchungselement für die Probe angefordert wird, nachdem sie von einer der Analysevorrichtungen in die entsprechende Transfermaschine befördert worden ist, die Betriebssteuereinheit dazu ausgelegt ist, die Beförderungsroute der Probe so zurückzusetzen, dass sie eine Analysevorrichtung enthält, die das zusätzliche Untersuchungselement analysieren kann, und dass die Probe gemäß der zurückgesetzten Beförderungsroute befördert wird;
**gekennzeichnet dadurch, dass**
das Probenbeförderungssystem ferner Informationslesevorrichtungen (40, 50, 90, 101, 111) umfasst, die dazu ausgelegt sind, die Probeninformationen der Probe zu lesen und die in jeder der Ladeeinheit (4), einer Abgabeeinheit (5) und den Transfermaschinen (9-11) angeordnet sind;
wenn eine Anomalie in einem Analyseergebnis der Probe an irgendeiner der mehreren Analysevorrichtungen (12-14) gefunden wird, die Analysevorrichtung dazu ausgelegt ist, das Anomalieergebnis an die Betriebssteuereinheit (21) zu melden, und die Betriebssteuereinheit dazu ausgelegt ist, die Beförderungsroute der Probe basierend auf dem gemeldeten Anomalieergebnis und basierend auf Probeninformationen, die von der in der mit der Analysevorrichtung verbundenen Transfermaschine angeordneten Informationslesevorrichtung gelesen wurden, zurückzusetzen, so dass die Probe, nachdem sie aus dem Analysevorrichtung in die Transfermaschine befördert wurde, gemäß der zurückgesetzten Beförderungsroute befördert wird; und
wenn die Probeninformationen der Probe, die das Anomalieergebnis aufweisen und die aus der Analysevorrichtung in die Transfermaschine befördert wurde, von der in der Transfermaschine angeordneten Informationslesevorrichtung nicht gelesen werden können, die Betriebssteuereinheit dazu ausgelegt ist, die Probe mit dem Anomalieergebnis von der Transfermaschine zur Ladeeinheit über die Beförderungslinie zu befördern, die Probeninformationen der Probe mit dem Anomalieergebnis von der in der Ladeeinheit angeordneten Informationslesevorrichtung zu lesen und die Beförderungsroute der Probe basierend auf den gelesenen Probeninformationen einzustellen.

2. Probenbeförderungsverfahren, umfassend:
Lesen von Probeninformationen einer von einer Ladeeinheit (4) geladenen Probe mit einer Informationslesevorrichtung (40);
Einstellen einer Beförderungsroute der Probe basierend auf den gelesenen Probeninformationen mit einer Betriebssteuereinheit (21);
Befördern der Probe zu einer oder mehreren von mehreren Analysevorrichtungen (12-14) durch eine Beförderungslinie (6-8) über eine entsprechende von mehreren Transfermaschinen (9-11), die mit jeder Analysevorrichtung und mit der Beförderungslinie verbunden sind, gemäß der eingestellten Beförderungsroute, um die Probe zu analysieren;
Zurücksetzen einer Beförderungsroute der Probe, so dass sie eine Analysevorrichtung enthält, die ein zusätzliches Untersuchungselement analysieren kann, wenn das zusätzliche Untersuchungselement für die Probe angefordert wird, nachdem sie nach der Analyse der Probe durch die Analysevorrichtung aus der Analysevorrichtung in die entsprechende Transfermaschine befördert wurde; und
Befördern der Probe gemäß der zurückgesetzten Beförderungsroute;
wobei, wenn eine Anomalie in einem Analyseergebnis der Probe an einer der mehreren Analysevorrichtungen (12-14) gefunden wird, die Analysevorrichtung das Anomalieergebnis an die Betriebssteuereinheit (21) meldet und die Betriebssteuereinheit die Beförderungsroute der Probe basierend auf dem gemeldeten Anomalieergebnis und basierend auf Probeninformationen, die von einer Informationslesevorrichtung (90, 101, 111) gelesen wurden, die in der mit der Analysevorrichtung verbundenen Transfermaschine angeordnet ist, zurücksetzt, so dass die Probe, nachdem sie aus der Analysevorrichtung in die Transfermaschine (91-11) befördert wurde, gemäß der zurückgesetzten Beförderungsroute befördert wird; und
wenn die Probeninformation der Probe mit dem Anomalieergebnis, die aus dem Analysevorrichtung in die Transfermaschine befördert wurde, von der in der Transfermaschine angeordneten Informationslesevorrichtung nicht gelesen werden kann, die Betriebssteuereinheit die Probe mit dem Anomalieergebnis von der Transfermaschine zur Ladeeinheit über die Beförderungslinie befördert, die Probeninformationen der Probe mit dem Anomalieergebnis durch die in der Probenladeinheit angeordnete Informationslesevorrichtung liest und eine Beförderungsroute der Probe basierend auf den gelesenen Probeninformationen einstellt.

## Revendications

1. Système de transport d'échantillon comprenant :
une unité (4) de chargement pour charger un échantillon ;
une ligne (6-8) de transport pour transporter l'échantillon dans les deux sens ;
une pluralité de dispositifs (12-14) d'analyse qui sont connectés à la ligne de transport et adaptés à analyser l'échantillon sur la base d'éléments d'examen de l'échantillon ;
une pluralité de machines (9-11) de transfert, chacune connectée à un correspondant de la pluralité de dispositifs d'analyse et à la ligne de transport et adaptée à transporter l'échantillon de la ligne de transport au dispositif d'analyse et du dispositif d'analyse à la ligne de transport,
une unité (3) de stockage pour recueillir et recevoir l'échantillon ; et
une unité (21) de commande de fonctionnement pour paramétrer un chemin de transport de l'échantillon avant que l'échantillon ne soit transporté par la ligne de transport,
dans lequel, lorsqu'un élément d'examen additionnel est demandé pour l'échantillon après qu'il a été transporté depuis un des dispositifs d'analyse jusque dans la machine de transfert correspondante, l'unité de commande de fonctionnement est adaptée à réinitialiser le chemin de transport de l'échantillon de façon à inclure un dispositif d'analyse qui peut analyser l'élément d'examen additionnel et de façon à ce que l'échantillon soit transporté conformément au chemin de transport réinitialisé ;
**caractérisé en ce que**
le système de transport d'échantillon comprend en outre des dispositifs (40, 50, 90, 101, 111) de lecture d'informations adaptés à lire les informations d'échantillon de l'échantillon et agencés dans chacune de l'unité (4) de chargement, d'une unité (5) de distribution, et des machines (9-11) de transfert ;
lorsqu'une anomalie est découverte dans un résultat d'analyse de l'échantillon dans un quelconque de la pluralité de dispositifs (12-14) d'analyse, le dispositif d'analyse est adapté à notifier le résultat d'anomalie à l'unité (21) de commande de fonctionnement, et l'unité de commande de fonctionnement est adaptée à réinitialiser le chemin de transport de l'échantillon sur la base du résultat d'anomalie notifié et sur la base d'informations d'échantillon lues par le dispositif de lecture d'informations agencé dans la machine de transfert connectée au dispositif d'analyse de façon à ce que l'échantillon, après qu'il a été transporté jusque dans la machine de transfert depuis le dispositif d'analyse, soit transporté conformément au chemin de transport réinitialisé ; et,
lorsque les informations d'échantillon de l'échantillon ayant le résultat d'anomalie et ayant été transporté jusque dans la machine de transfert depuis le dispositif d'analyse ne peuvent être lues par le dispositif de lecture d'informations agencé dans la machine de transfert, l'unité de commande de fonctionnement est adaptée à transporter l'échantillon ayant le résultat d'anomalie de la machine de transfert à l'unité de chargement par la ligne de transport, à lire les informations d'échantillon de l'échantillon ayant le résultat d'anomalie par le dispositif de lecture d'informations agencé dans l'unité de chargement, et à paramétrer le chemin de transport de l'échantillon sur la base des informations d'échantillon lues.

2. Procédé de transport d'échantillon comprenant :
la lecture d'informations d'échantillon d'un échantillon chargé depuis une unité (4) de chargement avec un dispositif (40) de lecture d'informations ;
le paramétrage d'un chemin de transport de l'échantillon sur la base des informations d'échantillon lues avec une unité (21) de commande de fonctionnement ;
le transport de l'échantillon jusqu'à un ou plusieurs parmi une pluralité de dispositifs (12-14) d'analyse par une ligne (6-8) de transport par l'intermédiaire d'une correspondante parmi une pluralité de machines (9-11) de transfert connectées à chaque dispositif d'analyse et à la ligne de transport conformément au chemin de transport paramétré pour analyser l'échantillon ;
la réinitialisation d'un chemin de transport de l'échantillon de façon à inclure un dispositif d'analyse qui peut analyser un élément d'examen additionnel, lorsque l'élément d'examen additionnel est demandé pour l'échantillon après qu'il a été transporté depuis le dispositif d'analyse jusque dans la machine de transfert correspondante après l'analyse de l'échantillon par le dispositif d'analyse ; et
le transport de l'échantillon conformément au chemin de transport réinitialisé ;
dans lequel, lorsqu'une anomalie est découverte dans un résultat d'analyse de l'échantillon dans un quelconque de la pluralité de dispositifs (12-14) d'analyse, le dispositif d'analyse notifie le résultat d'anomalie à l'unité (21) de commande de fonctionnement, et l'unité de commande de fonctionnement réinitialise le chemin de transport de l'échantillon sur la base du résultat d'anomalie notifié et sur la base d'informations d'échantillon lues par un dispositif (90, 101, 111) de lecture d'informations agencé dans la machine de transfert connectée au dispositif d'analyse de façon à ce que l'échantillon, après qu'il a été transporté jusque dans la machine (9-11) de transfert depuis le dispositif d'analyse, soit transporté conformément au chemin de transport réinitialisé ; et
lorsque les informations d'échantillon de l'échantillon ayant le résultat d'anomalie et ayant été transporté jusque dans la machine de transfert depuis le dispositif d'analyse ne peuvent être lues par le dispositif de lecture d'informations agencé dans la machine de transfert, l'unité de commande de fonctionnement transporte l'échantillon ayant le résultat d'anomalie de la machine de transfert à l'unité de chargement par la ligne de transport, lit les informations d'échantillon de l'échantillon ayant le résultat d'anomalie par le dispositif de lecture d'informations agencé dans l'unité de chargement d'échantillon, et paramètre un chemin de transport de l'échantillon sur la base des informations d'échantillon lues.
